# EUROPEAN PATENT APPLICATION

(11) **EP 2 474 323 A2**
(43) Date of publication of application: **11.07.2012**
(21) Application number: 12160896.2
(22) Date of filing: 24.03.2009
(51) Int. Cl.: A61K 45/06, A61P 31/00, A61K 31/506

(54) **Imidazoquinolines and pyrimidine derivatives as potent modulators of vegf-driven angiogenic processes**

(30) Priority: 26.03.2008 EP 08153311
(62) Divisional of application: 09726384.2
(71) Applicant: Novartis AG, 4056 Basel (CH)
(72) Inventor: GARCIA-ECHEVERRIA, Carolos, 4052 Basel (CH)
(74) Representative: Westberg, Marie Suzanne

(57) **Abstract**

The invention relates to the use of compounds of formula (I) or (II) 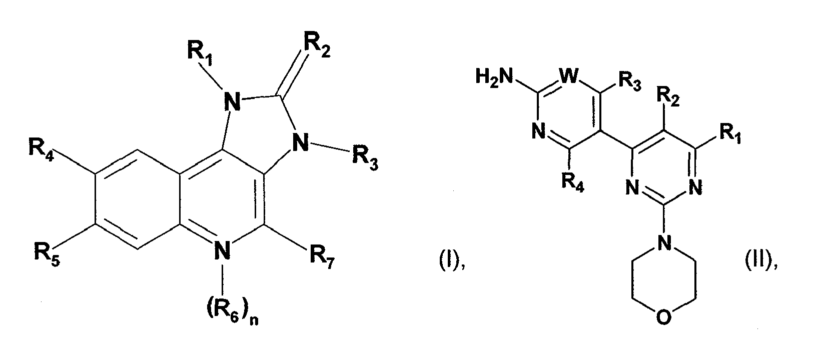 in the treatment of mammalian target of VEGF-driven angiogenic diseases, methods of use of said compounds in the treatment of said diseases in a warm-blooded animal, especially a human, pharmaceutical preparations comprising said compounds for the treatment of said diseases and said compounds for use in the treatment of said diseases.

## Description

The present invention relates to the use of specific imidazoquinoline and pyridine derivatives in the treatment of VEGF-dependent diseases or for the manufacture of pharmaceutical compositions for use in the treatment of said diseases, methods of use of specific imidazoquinoline and pyridine derivatives in the treatment of said diseases in a warm-blooded animal, especially a human, pharmaceutical preparations comprising specific imidazoquinoline and pyridine derivatives for the treatment of said diseases and specific imidazoquinoline and pyridine derivatives for use in the treatment of said diseases.

It has been found that specific imidazoquinoline and pyrimidine derivatives, which have been described in W02006/122806 and WO07/084786, respectively, to modulate the biological activity of PI3-kinases, are able to block the biological effects associated with the activation of VEGF receptors by their cognate ligands. Said compounds are thus useful for the treatment of VEGF-driven angiogenic diseases.

Syndromes with an established or potential molecular link to the VEGFR/VEGF axis are, for instance, described in "P. Carmeliet and RK Jain; Angiogenesis in cancer and other diseases, Nature 2000; 407: 249-257" and in SM Weiss and DA Cheresh; Pathophysiological consequences of VEGF-induced vascular permeability Nature 2005, 437:4697-50" which all are, including the references cited therein, hereby incorporated into the present application by reference, and are as follows:
- Rheumatoid arthritis
- Synovitis
- Bone and cartilage destruction
- Osteomyelitis
- Pannus Growth
- Osteophyte formation
- Hepatitis
- Pneumonia
- Glomerulonephritis
- Asthma
- Nasal polyps
- Transplantation
- Liver generation
- Retinopathy of prematurity
- Age macular degeneration
- Diabetic retinopathy
- Chroidal and other intraocular disorders
- Leukomalacia
- Thyroiditis
- Thyroid enlargement
- Lympopholiferative disorders
- Karposi's sarcoma
- Haematologic malignacies (e.g., haemangiomas)
- Obesity
- Spinal cord injury
- Acutemyocardial infarction
- Pulmonary, cerebral and retinal oedema
or further any combinations thereof.

Current anti-angiogenic therapies aim to target either the binding of ligands (by competition with an antagonist or by trapping of the endogenous ligand or by expression of a soluble form of the receptor) on their cognate receptors expressed at the surface of endothelial cells composing the blood vessels (e.g. VEGF binding on VEGFR1, 2 and 3); or by impeding on the activation of the receptors by using small molecular mass inhibitors that block the kinase activity of the tyrosine kinase receptor(s) (e.g. blockade of VEGFR1, 2 or 3 activation). Other strategies aiming at upregulating endogenous and natural inhibitor of VEGF induced pathway in endothelial cells, or at attacking the already existing vasculature with a VEGF-toxin conjugate have already been described. PI3K inhibitors exert their anti-angiogenic properties by blocking the propagation of VEGF induced signal when bound to VEGFR1, 2 or 3. The PI3K/Akt pathway is an important VEGFR downstream effector as it is required for survival and proliferation of endothelial cells in vitro and in vivo (HP Gerber et al, Vascular Endothelial Growth Factor Regulates Endothelial Cell Survival through the Phosphatidylinositol 3'-Kinase/Akt Signal Transduction Pathway. J Biol Chem 1998; 273(46):30336-30343; Y Fujio Y, and K Walsh . Akt Mediates Cytoprotection of Endothelial Cells by Vascular Endothelial Growth Factor in an Anchorage-dependent Manner. J Biol Chem 1999; 274(23):16349-16354; LE Benjamin, and E Keshet E. Conditional switching of vascular endothelial growth factor (VEGF) expression in tumors: Induction of endothelial cell shedding and regression of hemangioblastoma-like vessels by VEGF withdrawal. PNAS 1997; 94(16):8761-8766; TL Phung et al. Pathological angiogenesis is induced by sustained Akt signaling and inhibited by rapamycin. Cancer Cell 2006; 10(2):159-170). PI3K inhibitors have been shown to abrogate VEGF induced proliferation and survival ("V Dayanir et al. Identification of Tyrosine Residues in Vascular Endothelial Growth Factor Receptor-2/FLK-1 Involved in Activation of Phosphatidylinositol 3-Kinase and Cell Proliferation. J Biol Chem 2001; 276(21):17686-17692."), hence PI3K pathway interception is believed to have major effects on dysregulated vascular function (AK Olsson et al, Nature Review Molecular Cellular Biology, 2006; Vol 7, 359-371).

Specific imidazoquinoline derivatives which are suitable for the present invention, their preparation and suitable pharmaceutical formulations containing the same are described in WO2006/12280 and include compounds of formula I: 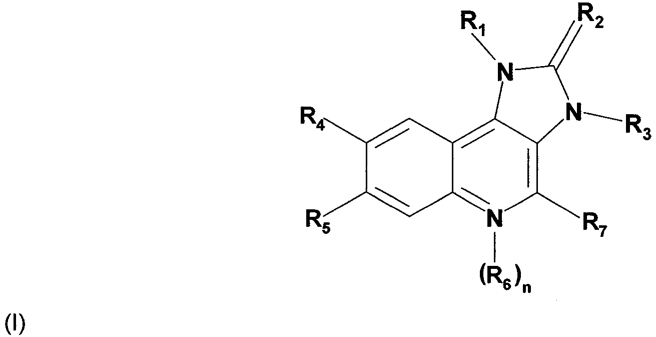 wherein
R₁ is naphthyl or phenyl wherein said phenyl is substituted by one or two substituents independently selected from the group consisting of Halogen; lower alkyl unsubstituted or substituted by halogen, cyano, imidazolyl or triazolyl; cycloalkyl; amino substituted by one or two substituents independently selected from the group consisting of lower alkyl, lower alkyl sulfonyl, lower alkoxy and lower alkoxy lower alkylamino; piperazinyl unsubstituted or substituted by one or two substituents independently selected from the group consisting of lower alkyl and lower alkyl sulfonyl; 2-oxo-pyrrolidinyl; lower alkoxy lower alkyl; imidazolyl; pyrazolyl; and triazolyl;
R₂ is O or S;
R₃ is lower alkyl;
R₄ is pyridyl unsubstituted or substituted by halogen, cyano, lower alkyl, lower alkoxy or piperazinyl unsubstituted or substituted by lower alkyl; pyrimidinyl unsubstituted or substituted by lower alkoxy; quinolinyl unsubstituted or substituted by halogen;
quinoxalinyl; or phenyl substituted with alkoxy
R₅ is hydrogen or halogen;
n is 0 or 1;
R₆ is oxido;
with the proviso that if n=1, the N-atom bearing the radical R₆ has a positive charge;
R₇ is hydrogen or amino;
or a tautomer thereof, or a pharmaceutically acceptable salt, or a hydrate or solvate thereof.

The radicals and symbols as used in the definition of a compound of formula I have the meanings as disclosed in WO2006/122806 which publication is hereby incorporated into the present application by reference.

A preferred compound of the present invention is a compound - described in WO2006/122806 - chosen from the group consisting of;
2-Methyl-2-[4-(3-methyl-2-oxo-8-pyridin-4-yl-2,3-dihydro-imidazo[4,5-c]quinolin-1-yl)-phenyl]-propionitrile;
2-Methyl-2-[4-(3-methyl-2-oxo-8-pyridin-3-yl-2,3-dihydro-imidazo[4,5-c]quinolin-1-yl)-phenyl]-propionitrile;
2-{4-[8-(6-Methoxy-pyridin-3-yl)-3-methyl-2-oxo-2,3-dihydro-imidazo[4,5-c]quinolin-1-yl]-phenyl}-2-methyl-propionitrile;
2-{4-[8-(5-Methoxy-pyridin-3-yl)-3-methyl-2-oxo-2,3-dihydro-imidazo[4,5-c]quinolin-1-yl]-phenyl}-2-methyl-propionitrile;
2-Methyl-2-{4-[3-methyl-2-oxo-8-(6-piperazin-1-yl-pyridin-3-yl)-2,3-dihydro-imidazo[4,5-c]quinolin-1-yl]-phenyl}-propionitrile;
2-Methyl-2-(4-{3-methyl-8-[2-(4-methyl-piperazin-1-yl)-pyridin-4-yl]-2-oxo-2,3-dihydro-imidazo[4,5-c]quinolin-1-yl}-phenyl)-propionitrile;
2-Methyl-2-[4-(3-methyl-2-oxo-8-quinolin-3-yl-2,3-dihydro-imidazo[4,5-c]quinolin-1-yl)-phenyl]-propionitrile;
2-{4-[8-(2-Fluoro-quinolin-3-yl)-3-methyl-2-oxo-2,3-dihydro-imidazo[4,5-c]quinolin-1-yl]-phenyl}-2-methyl-propionitrile;
2-Methyl-2-[4-(3-methyl-2-oxo-8-quinolin-6-yl-2,3-dihydro-imidazo[4,5-c]quinolin-1-yl)-phenyl]-propionitrile;
2-Methyl-2-[4-(3-methyl-2-oxo-8-quinolin-5-yl-2,3-dihydro-imidazo[4,5-c]quinolin-1-yl)-phenyl]-propionitrile;
2-Methyl-2-[4-(3-methyl-2-oxo-8-quinoxalin-6-yl-2,3-dihydro-im idazo[4,5-c]quinolin-1-yl)-phenyl]-propionitrile;
2-Ethyl-2-[4-(3-methyl-2-oxo-8-pyridin-3-yl-2,3-dihydro-imidazo[4,5-c]quinolin-1-yl)-phenyl]-butyronitrile;
2-Ethyl-2-[4-(3-methyl-2-oxo-8-quinolin-3-yl-2,3-dihydro-imidazo[4,5-c]quinolin-1-yl)-phenyl]-butyronitrile;
1-[3-Fluoro-4-(2-oxo-pyrrolidin-1-yl)-phenyl]-3-methyl-8-pyridin-3-yl-1,3-dihydro-imidazo[4,5-c]quinolin-2-one;
1-[3-Fluoro-4-(2-oxo-pyrrolidin-1-yl)-phenyl]-3-methyl-8-quinolin-3-yl-1,3-dihydro-imidazo[4,5-c]quinolin-2-one;
3-Methyl-1-[4-(2-oxo-pyrrolidin-1-yl)-phenyl]-8-pyridin-3-yl-1,3-dihydro-imidazo[4,5-c]quinolin-2-one;
3-Methyl-1-[4-(2-oxo-pyrrolidin-1-yl)-phenyl]-8-quinolin-3-yl-1,3-dihydro-imidazo[4,5-c]quinolin-2-one;
1-{4-[Bis-(2-methoxy-ethyl)-amino]-3-fluoro-phenyl}-3-methyl-8-pyridin-3-yl-1,3-dihydro-imidazo[4,5-c]quinolin-2-one;
1-{4-[Bis-(2-methoxy-ethyl)-amino]-3-fluoro-phenyl}-3-methyl-8-quinolin-3-yl-1,3-dihydro-imidazo[4,5-c]quinolin-2-one;
1-{4-[Bis-(2-methoxy-ethyl)-amino]-phenyl}-3-methyl-8-pyridin-3-yl-1,3-dihydro-imidazo[4,5-c]quinolin-2-one;
1-{4-[Bis-(2-methoxy-ethyl)-amino]-phenyl}-3-methyl-8-quinolin-3-yl-1,3-dihydro-imidazo[4,5-c]quinolin-2-one;
3-Methyl-1-naphthalen-2-yl-8-pyridin-3-yl-1,3-dihydro-imidazo[4, 5-c]quinolin-2-one;
3-Methyl-1-naphthalen-2-yl-8-quinolin-3-yl-1,3-dihydro-imidazo[4,5-c]quinolin-2-one;
1-(2-Chloro-phenyl)-3-methyl-8-pyridin-3-yl-1,3-dihydro-imidazo[4,5-c]quinolin-2-one;
1-(2-Chloro-phenyl)-3-methyl-8-quinolin-3-yl-1,3-dihydro-imidazo[4,5-c]quinolin-2-one;
3-Methyl-8-pyridin-3-yl-1-o-tolyl-1,3-dihydro-imidazo[4,5-c]quinolin-2-one;
3-Methyl-8-quinolin-3-yl-1-o-tolyl-1,3-dihydro-imidazo[4,5-c]quinolin-2-one;
1-(2-Ethyl-phenyl)-3-methyl-8-pyridin-3-yl-1,3-dihydro-imidazo[4,5-c]quinolin-2-one;
1-(2-Ethyl-phenyl)-3-methyl-8-quinolin-3-yl-1,3-dihydro-imidazo[4,5-c]quinolin-2-one;
3-Methyl-8-pyridin-3-yl-1-(2-trifluoromethyl-phenyl)-1,3-dihydro-imidazo[4,5-c]quinolin-2-one;
3-Methyl-8-quinolin-3-yl-1-(2-trifluoromethyl-phenyl)-1,3-dihydro-imidazo[4,5-c]quinolin-2-one;
1-(4-Fluoro-2-methyl-phenyl)-3-methyl-8-quinolin-3-yl-1,3-dihydro-imidazo[4,5-c]quinolin-2-one;
1-(4-Fluoro-2-methyl-phenyl)-3-methyl-8-quinolin-3-yl-1,3-dihydro-imidazo[4,5-c]quinolin-2-one;
1-(2-Chloro-4-fluoro-phenyl)-3-methyl-8-pyridin-3-yl-1,3-dihydro-imidazo[4,5-c]quinolin-2-one;
1-(2-Chloro-4-fluoro-phenyl)-3-methyl-8-quinolin-3-yl-1,3-dihydro-imidazo[4,5-c]quinolin-2-one;
1-(3-Chloro-phenyl)-3-methyl-8-pyridin-3-yl-1,3-dihydro-imidazo[4,5-c]quinolin-2-one;
1-(3-Chloro-phenyl)-3-methyl-8-quinolin-3-yl-1,3-dihydro-imidazo[4,5-c]quinolin-2-one;
3-Methyl-8-pyridin-3-yl-1-(3-trifluoromethyl-phenyl)-1,3-dihydro-imidazo[4,5-c]quinolin-2-one;
3-Methyl-8-quinolin-3-yl-1-(3-trifluoromethyl-phenyl)-1,3-dihydro-imidazo[4,5-c]quinolin-2-one;
1-(4-Methoxymethyl-phenyl)-3-methyl-8-pyridin-3-yl-1,3-dihydro-imidazo[4,5-c]quinolin-2-one;
1-(4-Methoxymethyl-phenyl)-3-methyl-8-quinofin-3-yl-1,3-dihydro-imidazo[4,5-c]quinolin-2-one;
1-[2-Chloro-4-(2-methoxy-ethyl)-phenyl]-3-methyl-8-pyridin-3-yl-1,3-dihydro-imidazo[4,5-c]quinolin-2-one;
1-[2-Chloro-4-(2-methoxy-ethyl)-phenyl]-3-methyl-8-quinolin-3-yl-1,3-dihydro-imidazo[4,5-c]quinolin-2-one;
1-[4-(2-Methoxy-ethyl)-phenyl]-3-methyl-8-quinolin-3-yl-1,3-dihydro-imidazo[4,5-c]quinolin-2-one;
1-[4-(2-Methoxy-ethyl)-phenyl]-3-methyl-8-pyridin-3-yl-1,3-dihydro-imidazo[4,5-c]quinolin-2-one;
2-Methyl-2-[4-(3-methyl-2-oxo-5-oxy-8-pyridin-3-yl-2,3-dihydro-imidazo[4,5-c]quinolin-1-yl)-phenyl]-propionitrile;
2-Methyl-2-[4-(3-methyl-2-oxo₋5-oxy-8-quinolin-3-yl-2,3-dihydro-imidazo[4,5-c]quinolin-1-yl)-phenyl]-propionitrile;
2-[4-(7-Fluoro-3-methyl-2-oxo-8-pyridin-3-yl-2,3-dihydro-imidazo[4,5-c]quinolin-1-yl)-phenyl]-2-methyl-propionitrile;
2-[4-(7-Fluoro-3-methyl-2-oxo-8-quinolin-3-yl-2,3-dihydro-imidazo[4,5-c]quinolin-1-yl)-phenyl]-2-methyl-propionitrile;
N-Methyl-N-[4-(3-methyl-2-oxo-8-pyridin-3-yl-2,3-dihydro-imidazo[4,5-c]quinolin-1-yl)-phenyl]-methanesulfonamide;
Methyl-[4-(3-methyl-2-oxo-8-pyridin-3-yl-2,3-dihydro-imidazo[4,5-c]quinolin-1-yl)-phenyl]-carbamic acid tert-butyl ester;
Ethanesulfonic acid methyl-[4-(3-methyl-2-oxo-8-pyridin-3-yl-2,3-dihydro-imidazo[4,5-c]quinolin-1-yl)-phenyl]-amide;
Ethanesulfonic acid methyl-[4-(3-methyl-2-oxo-8-quinolin-3-yl-2,3-dihydro-imidazo[4,5-c]quinolin-1-yl)-phenyl]-amide;
N-Ethyl-N-[4-(3-methyl-2-oxo-8-pyridin-3-yl-2,3-dihydro-imidazo[4,5-c]quinolin-1-yl)-phenyl]-methanesulfonamide;
N-Ethyl-N-[4-(3-methyl-2-oxo-8-quinolin-3-yl-2,3-dihydro-imidazo[4,5-c]quinolin-1-yl)-phenyl]-methanesulfonamide;
2-[4-(3-Ethyl-2-oxo-8-pyridin-3-yl-2,3-dihydro-imidazo[4,5-c]quinolin-1-yl)-phenyl]-2-methylpropionitrile;
1-[3-Fluoro-4-(4-methanesulfonyl-piperazin-1-yl)-phenyl]-3-methyl-8-quinolin-3-yl-1,3-dihydro-imidazo[4,5-c]quinolin-2-one;
1-[3-Fluoro-4-(4-methanesulfonyl-piperazin-1-yl)-phenyl]-3-methyl-8-pyridin-3-yl-1,3-dihydro-imidazo[4,5-c]quinolin-2-one;
1-(3-Fluoro-4-piperazin-1-yl-phenyl)-3-methyl-8-quinolin-3-yl-1,3-dihydro-imidazo[4,5-c]quinolin-2-one;
1-(3-Fluoro-4-piperazin-1-yl-phenyl)-3-methyl-8-quinolin-3-yl-1,3-dihydro-imidazo[4,5-c]quinolin-2-one;
1-(3-Fluoro-4-piperazin-1-yl-phenyl)-3-methyl-8-quinolin-3-yl-1,3-dihydro-imidazo[4,5-c]quinolin-2-one;
1-(3-Fluoro-4-piperazin-1-yl-phenyl)-3-methyl-8-quinolin-3-yl-1,3-dihydro-imidazo[4,5-c]quinolin-2-one;
1-[2-Chloro-4-(4-methyl-piperazin-1-yl)-phenyl]-3-methyl-8-quinolin-3-yl-1,3-dihydro-imidazo[4,5-c]quinolin-2-one;
1-[2-Chloro-4-(4-methyl-piperazin-1-yl)-phenyl]-3-methyl-8-pyridin-3-yl-1,3-dihydro-imidazo[4,5-c]quinolin-2-one;
1-[3-Chloro-4-(4-methyl-piperazin-1-yl-phenyl]-3-methyl-8-quinolin-3-yl-1,3-dihydro-imidazo[4,5-c]quinolin-2-one;
1-[3-Chloro-4-(4-methyl-piperazin-1-yl-phenyl)]-3-methyl-8-pyridin-3-yl-1,3-dihydro-imidazo[4,5-c]quinolin-2-one;
1-(4-Imidazol-1-yl-2-methyl-phenyl)-3-methyl-8-quinolin-3-yl-1,3-dihydro-imidazo[4,5-c]quinolin-2-one;
1-(4-Imidazol-1-yl-2-methyl-phenyl)-3-methyl-8-pyridin-3-yl-1,3-dihydro-imidazo[4,5-c]quinolin-2-one;
3-Methyl-1-(4-pyrazol-1-yl-phenyl)-8-quinolin-3-yl-1,3-dihydro-imidazo[4,5-c]quinolin-2-one;
3-Methyl-1-(4-pyrazol-1-yl-phenyl)-8-pyridin-3-yl-1,3-dihydro-imidazo[4,5-c]quinolin-2-one;
3-Methyl-8-quinolin-3-yl-1-(4-[1,2,4]triazol-1-yl-phenyl)-1,3-dihydro-imidazo[4,5-c]quinolin-2-one;
3-Methyl-8-pyridin-3-yl-1-(4-[1,2,4]triazol-1-yl-phenyl)-1,3-dihydro-imidazo[4,5-c]quinolin-2-one;
3-Methyl-1-[4-(4-methyl-piperazin-1-yl)-3-trifluoromethyl-phenyl]-8-quinolin-3-yl-1,3-dihydro-imidazo[4,5-c]quinolin-2-one;
3-Methyl-1-[4-(4-methyl-piperazin-1-yl)-3-trifluoromethyl-phenyl]-8-pyridin-3-yl-1,3-dihydro-imidazo[4,5-c]quinolin-2-one;
1-(3-Chloro-4-piperazin-1-yl-phenyl)-3-methyl-8-quinolin-3-yl-1,3-dihydro-imidazo[4,5-c]quinolin-2-one;
1-(3-Chloro-4-piperazin-1-yl-phenyl)-3-methyl-8-pyridin-3-yl-1,3-dihydro-imidazo[4,5-c]quinolin-2-one;
1-(3-Chloro-4-piperazin-1-yl-phenyl)-8-(6-methoxy-pyridin-3-yl)-3-methyl-1,3-dihydro-imidazo[4,5-c]quinolin-2-one;
1-(3-Chloro-4-piperazin-1-yl-phenyl)-8-(5-methoxy-pyridin-3-yl)-3-methyl-1,3-dihydro-imidazo[4,5-c]quinolin-2-one;
8-(6-Methoxy-pyridin-3-yl)-3-methyl-1-[4-(4-methyl-piperazin-1-yl)-3-trifluoromethyl-phenyl]-1,3-dihydro-imidazo[4,5-c]quinolin-2-one;
8-(5-Methoxy-pyridin-3-yl)-3-methyl-1-[4-(4-methyl-piperazin-1-yl)-3-trifluoromethyl-phenyl]-1,3-dihydro-imidazo[4,5-c]quinolin-2-one;
1-[2-Chloro-4-(4-methyl-piperazin-1-yl)-phenyl]-8-(6-methoxy-pyridin-3-yl)-3-methyl-1,3-dihydro-imidazo[4,5-c]quinolin-2-one;
1-[2-Chloro-4-(4-methyl-piperazin-1-yl)-phenyl]-8-(5-methoxy-pyridin-3-yl)-3-methyl-1,3-dihydro-imidazo[4,5-c]quinolin-2-one;
1-(3-Chloro-4-piperazin-1-yl-phenyl)-3-methyl-8-quinoxalin-6-yl-1,3-dihydro-imidazo[4,5-c]quinolin-2-one;
3-Methyl-1-(4-piperazin-1-yl-3-trifluoromethyl-phenyl)-8-quinolin-3-yl-1,3-dihydro-imidazo[4,5-c]quinolin-2-one;
3-Methyl-1-(4-piperazin-1-yl-3-trifluoromethyl-phenyl)-8-pyridin-3-yl-1,3-dihydro-imidazo[4,5-c]quinolin-2-one;
8-(6-Methoxy-pyridin-3-yl)-3-methyl-1-(4-piperazin-1-yl-3-trifluoromethyl-phenyl)-1,3-dihydro-imidazo[4,5-c]quinolin-2-one;
8-(5-Methoxy-pyridin-3-yl)-3-methyl-1-(4-piperazin-1-yl-3-trifluoromethyl-phenyl)-1,3-dihydro-imidazo[4,5-c]quinolin-2-one;
3-Methyl-1-(4-piperazin-1-yl-3-trifluoromethyl-phenyl)-8-quinoxalin-6-yl-1,3-dihydro-imidazo[4,5-c]quinolin-2-one;
1-[3-Chloro-4-(*cis*-3,5-dimethyl-piperazin-1-yl)-phenyl]-3-methyl-8-pyridin-3-yl-1,3-dihydro-imidazo[4,5-c]quinolin-2-one;
1-[3-Chloro-4-(*cis*-3,5-dimethyl-piperazin-1-yl)-phenyl]-3-methyl-8-quinolin-3-yl-1,3-dihydro-imidazo[4,5-c]quinolin-2-one;
1-[3-Chloro-4-(4-ethyl-piperazin-1-yl)-phenyl]-3-methyl-8-pyridin-3-yl-1,3-dihydro-imidazo[4,5-c]quinolin-2-one;
1-[3-Chloro-4-(4-ethyl-piperazin-1-yl)-phenyl]-3-methyl-8-quinolin-3-yl-1,3-dihydro-imidazo[4,5-c]quinolin-2-one;
1-[3-Chloro-4-(4-isopropyl-piperazin-1-yl)-phenyl]-3-methyl-8-pyridin-3-yl-1,3-dihydro-imidazo[4,5-c]quinolin-2-one;
1-[3-Chloro-4-(4-isopropyl-piperazin-1-yl)-phenyl]-3-methyl-8-quinolin-3-yl-1,3-dihydro-imidazo[4,5-c]quinolin-2-one;
1-[3-Chloro-4-(4-isopropyl-piperazin-1-yl)-phenyl]-3-methyl-8-quinolin-3-yl-1,3-dihydro-imidazo[4,5-c]quinolin-2-one;
1-[3-Chloro-4-(4-isopropyl-piperazin-1-yl)-phenyl]-3-methyl-8-quinolin-3-yl-1,3-dihydro-imidazo[4,5-c]quinolin-2-one;
1-[4-(4-Ethyl-piperazin-1-yl)-3-trifluoromethyl-phenyl]-3-methyl-8-pyridin-3-yl-1,3-dihydro-imidazo[4,5-c]quinolin-2-one;
1-[4-(4-Ethyl-piperazin-1-yl)-3-trifluoromethyl-phenyl]-3-methyl-8-quinolin-3-yl-1,3-dihydro-imidazo[4,5-c]quinolin-2-one;
1-[4-(4-Ethyl-piperazin-1-yl)-3-trifluoromethyl-phenyl]-3-methyl-8-pyridin-3-yl-1,3-dihydro-imidazo[4,5-c]quinolin-2-one;
1-[4-(4-Ethyl-piperazin-1-yl)-3-trifluoromethyl-phenyl]-3-methyl-8-quinolin-3-yl-1,3-dihydro-imidazo[4,5-c]quinolin-2-one;
3-Methyl-8-(6-piperazin-1-yl-pyridin-3-yl)-1-(3-trifluoromethyl-phenyl)-1,3-dihydro-imidazo[4,5-c]quinolin-2-one;
8-(6-Methoxy-pyridin-3-yl)-3-methyl-1-(3-trifluoromethyl-phenyl)-1,3-dihydro-imidazo[4,5-c]quinolin-2-one;
8-(6-Methoxy-pyridin-3-yl)-3-methyl-1-(3-trifluoromethyl-phenyl)-1,3-dihydro-imidazo[4,5-c]quinolin-2-one;
1-(3-Chloro-4-imidazol-1-yl-phenyl)-3-methyl-8-pyridin-3-yl-1,3-dihydro-imidazo[4,5-c]quinolin-2-one;
1-(3-Chloro-4-imidazol-1-yl-phenyl)-3-methyl-8-quinolin-3-yl-1,3-dihydro-imidazo[4,5-c]quinolin-2-one;
2-Methyl-2-[4-(3-methyl-8-quinolin-3-yl-2-thioxo-2,3-dihydro-imidazo[4,5-c]quinolin-1-yl)-phenyl]-propionitrile;
2-Methyl-2-{4-[3-methyl-8-(2-methyl-pyridin-4-yl)-2-oxo-2,3-dihydro-imidazo[4,5-c]quinolin-1-yl]-phenyl}-propionitrile;
5-{1-[4-(Cyano-dimethyl-methyl)-phenyl]-3-methyl-2-oxo-2,3-dihydro-1H-imidazo[4,5-c]quinolin-8-yl}-pyridine-2-carbonitrile;
2-[4-(4-Amino-3-methyl-2-oxo-8-quinolin-3-yl-2,3-dihydro-imidazo[4,5-c]quinolin-1-yl)-phenyl]-2-methyl-propionitrile;
1-[4-(3-Methyl-2-oxo-8-pyridin-3-yl-2,3-dihydro-imidazo[4,5-c]quinolin-1-yl)-phenyl]-cyclopropanecarbonitrile;
1-[4-(3-Methyl-2-oxo-8-quinolin-3-yl-2,3-dihydro-imidazo[4,5-c]quinolin-1-yl)-phenyl]-cyclopropanecarbonitrile;
1-{4-[8-(6-Methoxy-pyridin-3-yl)-3-methyl-2-oxo-2,3-dihydro-imidazo[4,5-c]quinolin-1-yl]-phenyl}-cyclopropanecarbonitrile;
1-[3-Chloro-4-(4-methyl-piperazin-1-yl)-phenyl]-8-(6-methoxy-pyridin-3-yl)-3-methyl-1,3-dihydro-imidazo[4,5-c]quinolin-2-one;
1-[3-Chloro-4-(4-methyl-piperazin-1-yl)-phenyl]-8-(5-methoxy-pyridin-3-yl)-3-methyl-1,3-dihydro-imidazo[4,5-c]quinolin-2-one;
1-[3-Chloro-4-(4-methyl-piperazin-1-yl)-phenyl]-3-methyl-8-quinoxalin-6-yl-1,3-dihydro-imidazo[4,5-c]quinolin-2-one;
1-(3-Chloro-4-piperazin-1-yl-phenyl)-8-(2-methoxy-pyrimidin-5-yl)-3-methyl-1,3-dihydro-imidazo[4,5-c]quinolin-2-one;
1-(3-Chloro-4-piperazin-1-yl-phenyl)-3-methyl-8-pyrimidin-5-yl-1,3-dihydro-imidazo[4,5-c]quinolin-2-one;
1-(3-Chloro-4-piperazin-1-yl-phenyl)-8-(2-methoxy-pyrimidin-5-yl)-3-methyl-1,3-dihydro-imidazo[4,5-c]quinolin-2-one;
1-(3-Chloro-4-piperazin-1-yl-phenyl)-3-methyl-8-pyrimidin-5-yl-1,3-dihydro-imidazo[4,5-c]quinolin-2-one;
1-(3-Chloro-4-piperazin-1-yl-phenyl)-3-methyl-8-(2-methyl-pyridin-4-yl)-1,3-dihydro-imidazo[4,5-c]quinolin-2-one;
1-[3-Chloro-4-(*cis*-3,5-dimethyl-piperazin-1-yl)-phenyl]-8-(6-methoxy-pyridin-3-yl)-3-methyl-1,3-dihydro-imidazo[4,5-c]quinolin-2-one;
1-[3-Chloro-4-(*cis*-3,5-dimethyl-piperazin-1-yl)-phenyl]-8-(5-methoxy-pyridin-3-yl)-3-methyl-1,3-dihydro-imidazo[4,5-c]quinolin-2-one;
1-[4-(*cis*-3,5-Dimethyl-piperazin-1-yl)-3-trifluoromethyl-phenyl]-8-(6-methoxy-pyridin-3-yl)-3-methyl-1,3-dihydro-imidazo[4,5-c]quinolin-2-one;
1-[4-(*cis*-3,5-Dimethyl-piperazin-1-yl)-3-trifluoromethyl-phenyl]-8-(5-methoxy-pyridin-3-yl)-3-methyl-1,3-dihydro-imidazo[4,5-c]quinolin-2-one;
8-(2-Methoxy-pyrimidin-5-yl)-3-methyl-1-(4-piperazin-1-yl-3-trifluoromethyl-phenyl)-1,3-dihydro-imidazo[4,5-c]quinolin-2-one;
3-Methyl-1-(4-piperazin-1-yl-3-trifluoromethyl-phenyl)-8-pyrimidin-5-yl-1,3-dihydro-imidazo[4,5-c]quinolin-2-one;
5-[3-Methyl-2-oxo-1-(4-piperazin-1-yl-3-trifluoromethyl-phenyl)-2,3-dihydro-1H-imidazo[4,5-c]quinolin-8-yl]-pyridine-2-carbonitrile;
3-Methyl-8-(2-methyl-pyridin-4-yl)-1-(4-piperazin-1-yl-3-trifluoromethyl-phenyl)-1,3-dihydro-imidazo[4,5-c]quinolin-2-one;
8-(3,4-Dimethoxy-phenyl)-3-methyl-1-(4-piperazin-1-yl-3-trifluoromethyl-phenyl)-1,3-dihydro-imidazo[4,5-c]quinolin-2-one;
3-Methyl-8-pyridin-3-yl-1-(4-[1,2,4]triazol-1-yl-3-trifluoromethyl-phenyl)-1,3-dihydro-imidazo[4,5-c]quinolin-2-one;
3-Methyl-8-quinolin-3-yl-1-(4-[1,2,4]triazol-1-yl-3-trifluoromethyl-phenyl)-1,3-dihydro-imidazo[4,5-c]quinolin-2-one;
8-(6-Methoxy-pyridin-3-yl)-3-methyl-1-(4-[1,2,4]triazol-1-yl-3-trifluoromethyl-phenyl)-1,3-dihydro-imidazo[4,5-c]quinolin-2-one;
8-(5-Methoxy-pyridin-3-yl)-3-methyl-1-(4-[1,2,4]triazol-1-yl-3-trifluoromethyl-phenyl)-1,3-dihydro-imidazo[4,5-c]quinolin-2-one;
5-[3-Methyl-2-oxo-1-(4-[1,2,4]triazol-1-yl-3-trifluoromethyl-phenyl)-2,3-dihydro-1H-imidazo[4,5-c]quinolin-8-yl]-pyridine-2-carbonitrile;
8-(6-Fluoro-pyridin-3-yl)-3-methyl-1-(4-[1,2,4]triazol-1-yl-3-trifluoromethyl-phenyl)-1,3-dihydro-imidazo[4,5-c]quinolin-2-one;
8-(2,6-Dimethoxy-pyridin-3-yl)-3-methyl-1-(4-[1,2,4]triazol-1-yl-3-trifluoromethyl-phenyl)-1,3-dihydro-imidazo[4,5-c]quinolin-2-one;
3-Methyl-8-pyrimidin-5-yl-1-(4-[1,2,4]triazol-1-yl-3-trifluoromethyl-phenyl)-1,3-dihydro-imidazo[4,5-c]quinolin-2-one;
8-(2-Methoxy-pyrimidin-5-yl)-3-methyl-1-(4-[1,2,4]triazol-1-yl-3-trifluoromethyl-phenyl)-1,3-dihydro-imidazo[4,5-c]quinolin-2-one;
8-(2,4-Dimethoxy-pyrimidin-5-yl)-3-methyl-1-(4-[1,2,4]triazol-1-yl-3-trifluoromethyl-phenyl)-1,3-dihydro-imidazo[4,5-c]quinolin-2-one;
3-Methyl-1-(4-pyrazol-1-yl-3-trifluoromethyl-phenyl)-8-pyridin-3-yl-1,3-dihydro-imidazo[4,5-c]quinolin-2-one;
3-Methyl-1-(4-pyrazol-1-yl-3-trifluoromethyl-phenyl)-8-quinolin-3-yl-1,3-dihydro-imidazo[4,5-c]quinolin-2-one;
8-(6-Methoxy-pyridin-3-yl)-3-methyl-1-(4-pyrazol-1-yl-3-trifluoromethyl-phenyl)-1,3-dihydro-imidazo[4,5-c]quinolin-2-one;
8-(5-Methoxy-pyridin-3-yl)-3-methyl-1-(4-pyrazol-1-yl-3-trifluoromethyl-phenyl)-1,3-dihydro-imidazo[4,5-c]quinolin-2-one;
1-(3-Chloro-4-[1,2,4]triazol-1-yl-phenyl)-3-methyl-8-pyridin-3-yl-1,3-dihydro-imidazo[4,5-c]quinolin-2-one;
1-(3-Chloro-4-[1,2,4]triazol-1-yl-phenyl)-3-methyl-8-quinolin-3-yl-1,3-dihydro-imidazo[4,5-c]quinolin-2-one;
1-(4-Imidazol-1-yl-3-trifluoromethyl-phenyl)-3-methyl-8-pyridin-3-yl-1,3-dihydro-imidazo[4,5-c]quinolin-2-one;
1-(4-Imidazol-1-yl-3-trifluoromethyl-phenyl)-3-methyl-8-quinolin-3-yl-1,3-dihydro-imidazo[4,5-c]quinolin-2-one;
1-(4-Imidazol-1-yl-3-trifluoromethyl-phenyl)-8-(6-methoxy-pyridin-3-yl)-3-methyl-1,3-dihydro-imidazo[4,5-c]quinolin-2-one;
1-(4-Imidazol-1-yl-3-trifluoromethyl-phenyl)-8-(5-methoxy-pyridin-3-yl)-3-methyl-1,3-dihydro-imidazo[4,5-c]quinolin-2-one;
3-Methyl-8-pyridin-3-yl-1-(4-[1,2,4]triazol-1-ylmethyl-phenyl)-1,3-dihydro-imidazo[4,5-c]quinolin-2-one;
3-Methyl-8-quinolin-3-yl-1-(4-[1,2,4]triazol-1-ylmethyl-phenyl)-1,3-dihydro-imidazo[4,5-c]quinolin-2-one;
1-(4-Imidazol-1-ylmethyl-phenyl)-3-methyl-8-pyridin-3-yl-1,3-dihydro-imidazo[4,5-c]quinolin-2-one; and
1-(4-Imidazol-1-ylmethyl-phenyl)-3-methyl-8-quinolin-3-yl-1,3-dihydro-imidazo[4,5-c]quinolin-2-one;
or a tautomer thereof, or a pharmaceutically acceptable salt, or a hydrate or solvate thereof.

A particularly preferred compound of the present invention is 2-methyl-2-[4-(3-methyl-2-oxo-8-quinolin-3-yl-2,3-dihydro-imidazo[4,5-c]quinolin-1-yl)-phenyl]-propionitrile. The synthesis of 2-methyl-2-[4-(3-methyl-2-oxo-8-quinolin-3-yl-2,3-dihydro-imidazo[4,5-c]quinolin-1-yl)-phenyl]-propionitrile is for instance described in WO2006/122806 as Example 1 (compound **A).**

Another particularly preferred compound of the present invention is 8-(6-methoxy-pyridin-3-yl)-3-methyl-1-(4-piperazin-1-yl-3-trifluoromethyl-phenyl)-1,3-dihydro-imidazo[4,5-c]quinolin-2-one. The synthesis of 8-(6-methoxy-pyridin-3-yl)-3-methyl-1-(4-piperazin-1-yl-3-trifluoromethyl-phenyl)-1,3-dihydro-imidazo[4,5-c]quinolin-2-one is for instance described in WO2006/122806 as Example 86 (compound B).

Specific pyrimidine derivatives which are suitable for the present invention, their preparation and suitable pharmaceutical formulations containing the same are described in WO07/084786 and include compounds of formula II 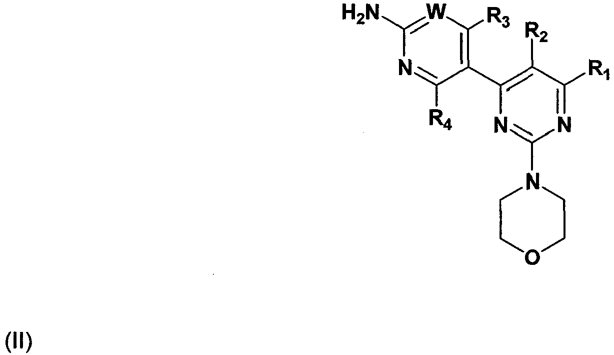 or a stereoisomer, tautomer, or pharmaceutically acceptable salt thereof wherein W is C, R_{w} or N, wherein R_{w} is selected from the group consisting of:
(1) hydrogen,
(2) cyano,
(3) halogen,
(4) methyl,
(5) trifluoromethyl,
(6) sulfonamido;
R₁ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₁ₐ,
(13) -CO₂R₁ₐ,
(14) -CONR₁ₐR_{1b},
(15) -NR₁ₐR_{1b},
(16) -NR₁ₐCOR_{1b},
(17) -NR₁ₐSO₂R_{1b},
(18) -OCOR₁ₐ,
(19) -OR₁ₐ,
(20) -SR₁ₐ,
(21) -SOR₁ₐ,
(22) -SO₂R₁ₐ, and
(23) -SO₂NR₁ₐR_{1b},
wherein R₁ₐ, and R_{1b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl;
R₂ is selected from the group consisting
(1) hydrogen,
(2) cyano,
(3) nitro,
(4) halogen,
(5) hydroxy,
(6) amino,
(7) substituted and unsubstituted alkyl,
(8) -COR₂ₐ, and
(9) -NR₂ₐCOR_{2b},
wherein R₂ₐ, and R_{2b} are independently selected from the group consisting of
(a) hydrogen, and
(b) substituted or unsubstituted alkyl;
R₃ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₃ₐ,
(13) -NR₃ₐR_{3b},
(14) -NR₃ₐCOR_{3b},
(15) -NR₃ₐSO₂R_{3b},
(16) -OR₃ₐ,
(17) -SR₃ₐ,
(18) -SOR₃ₐ,
(19) -SO₂R₃ₐ, and
(20) -SO₂NR₃ₐR_{3b},
wherein R₃ₐ, and R_{3b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl; and
R₄ is selected from the group consisting of
(1) hydrogen, and
(2) halogen.

The radicals and symbols as used in the definition of a compound of formula II have the meanings as disclosed in WO07/084786 which publication is hereby incorporated into the present application by reference.

A preferred compound of the present invention is a compound which is specifically described in WO07/084786. A particularly preferred compound of the present invention is 5-(2,6-di-morpholin-4-yl-pyrimidin-4-yl)-4-trifluoromethyl-pyridin-2-ylamine (Compound C). The synthesis of 5-(2,6-di-morpholin-4-yl-pyrimidin-4-yl)-4-trifluoromethyl-pyridin-2-ylamine is described in WO07/084786 as Example 10.

According to the present invention the treatment of:
- Rheumatoid arthritis
- Synovitis
- Bone and cartilage destruction
- Osteomyelitis
- Pannus Growth
- Osteophyte formation
- Hepatitis
- Pneumonia
- Glomerulonephritis
- Asthma
- Nasal polyps
- Transplantation
- Liver generation
- Retinopathy of prematurity
- Age macular degeneration
- Diabetic retinopathy
- Chroidal and other intraocular disorders
- Leukomalacia
- Thyroiditis
- Thyroid enlargement
- Lympopholiferative disorders
- Karposi's sarcoma
- Haematologic malignacies (e.g., haemangiomas)
- Obesity
- Spinal cord injury
- Acutemyocardial infarction
- Pulmonary, cerebral and retinal oedema
Or any further combination thereof.
with compounds of formula I and II are especially preferred:

In particular, the present invention relates to a method of treating a VEGF-driven angiogenic disease comprising administering a therapeutically effective amount of a specific imidazoquinoline derivative of formula I, especially preferred 2-methyl-2-[4-(3-methyl-2-oxo-8-quinolin-3-yl-2,3-dihydro-imidazo[4,5-c]quinolin-1-yl)-phenyl]-propionitrile (compound A) or 8-(6-methoxy-pyridin-3-yl)-3-methyl-1-(4-piperazin-1-yl-3-trifluoromethyl-phenyl)-1,3-dihydro-imidazo[4,5-c]quinolin-2-one (compound B) or a specific pyridine derivative of formula II, especially preferrred 5-(2,6-di-morpholin-4-yl-pyrimidin-4-yl)-4-trifluoromethyl-pyridin-2-ylamine (Compound C), to a warm-blooded animal in need thereof.

Furthermore, the present invention relates to the use of a specific imidazoquinoline derivative of formula I, especially preferred 2-methyl-2-[4-(3-methyl-2-oxo-8-quinolin-3-yl-2,3-dihydro-imidazo[4,5-c]quinolin-1-yl)-phenyl]-propionitrile (compound A) or 8-(6-methoxy-pyridin-3-yl)-3-methyl-1-(4-piperazin-1-yl-3-trifluoromethyl-phenyl)-1,3-dihydro-imidazo[4,5-c]quinolin-2-one (compound B) or a specific pyridine derivative of formula II, especially preferred 5-(2,6-di-morpholin-4-yl-pyrimidin-4-yl)-4-trifluoromethyl-pyridin-2-ylamine (Compound C) for the manufacture of a pharmaceutical preparation for the treatment of a VEGF-driven angiogenic disease or malignancy or a disease that has acquired resistance to agents that target VEGF and/or VEGFR family members.

The resistance to the treatment with a VEGF and/or VEGFR modulator can be acquired during treatment with said VEGF and/or VEGFR modulator by different mechanisms

In particular, the present invention relates to the treatment of a disease or malignancy that is dependent on VEGF or has aquired resistance during treatment with a modulator of the VEGF/VEGFR axis, with compounds of formula I, especially preferred 2-methyl-2-[4-(3-methyl-2-oxo-8-quinolin-3-yl-2,3-dihydro-imidazo[4,5-c]quinolin-1-yl)-phenyl]-propionitrile (compound A) or 8-(6-methoxy-pyridin-3-yl)-3-methyl-1-(4-piperazin-1-yl-3-trifluoromethylphenyl)-1,3-dihydro-imidazo[4,5-c]quinolin-2-one (compound B) or of formula II, especially preferred 5-(2,6-di-morpholin-4-yl-pyrimidin-4-yl)-4-trifluoromethyl-pyridin-2-ylamine (Compound C) or a pharmaceutically acceptable salt thereof. Possible agents that target the VEGF/VEGFR axis are, for instance Bevacizumab, Ranibizumab, AVE0005, HuMV833, 2C3, CBO-P11, Sutent, Sorafenib, Vatalanib, Zactima, Midostaurin, Angiozyme, AG-013736, Lestautinib, CP-547,632, CEP-7055, KRN633, NVP-AEE788, IMC-1211, ZK260253, Semaxanib, E-7107, AS-3, Cand5 and PTC-299.

A compound of the formula (I) or (II) may also be used for the treatment of VEGF-driven angiogenic dieseases in combination with other active compounds for instance the combination partners as disclosed in W02006/122806 and WO07/084786, more preferred VEGF or VEGFR targeting agents such as, and without limitation instance anti-VEGF Bevacizumab, anti-VEGF, Ranibizumab AVE0005, anti-VEGF HuMV833, anti-VEGF 2C3, anti-VEGF CBO-P11, Sutent, Sorafenib, Vatalanib, Zactima, Midostaurin, Angiozyme, AG-013736, Lestautinib, CP-547,632, CEP-7055, KRN633, NVP-AEE788, IMC-1211, ZK260253, Semaxanib, E-7107, AS-3, Cand5 and PTC-299; and the HSP90 inhibitors CNF1010, CNF2024, tanespimycinm, alvespimycin, IP1504, SNX5422 and NVP-AUY922.

By "combination" according to the invention, there is meant either a fixed combination in one dosage unit form, or a kit of parts for the combined administration where a compound of the formula (I) and a combination partner may be administered independently at the same time or separately within time intervals that especially allow that the combination partners show a cooperative, e.g. synergistic effect.

A compound of formula I can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The compounds of the invention may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising a compound of the invention in association with at least one pharmaceutical acceptable carrier or diluent may be manufactured in conventional manner by mixing with a pharmaceutically acceptable carrier or diluent.

The pharmaceutical compositions are comprising an amount effective in the treatment of one of the above-mentioned disorders, of a compound of formula I or an N-oxide or a tautomer thereof together with pharmaceutically acceptable carriers that are suitable for topical, enteral, for example oral or rectal, or parenteral administration and that may be inorganic or organic, solid or liquid. There are pharmaceutical compositions used for oral administration especially tablets or gelatin capsules that comprise the active ingredient together with diluents, for example lactose, dextrose, mannitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilisers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectioning, dissolving or lyophilising processes, and comprise approximately from 1% to 99%, especially from approximately 1% to approximately 20%, active ingredient(s).

### Brief Description of the Drawings:

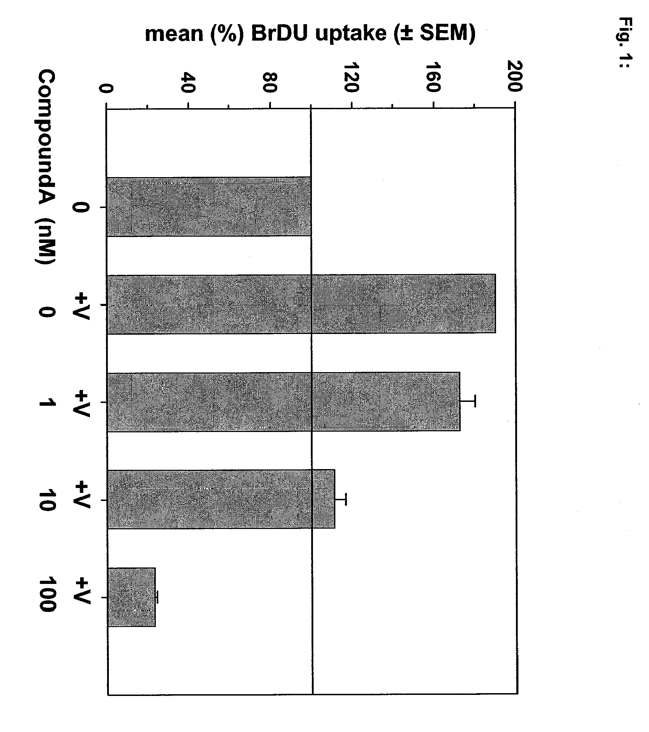

Fig. 1 shows the effect of compound A on VEGF induced proliferation. HUVEC cells were seeded, starved in low serum with (V) or without (0) VEGF- and BrDU-containing medium and incubated with increasing concentrations of compound A for a period of 24 h. Endothelial cell proliferation was measured by quantification of incorporated BrDU. Four independent experiments were performed with n = 3 wells per group*, p = < 0.05 (ANOVA-Dunnet's) over baseline, VEGF treated controls (represented by the horizontal line). X-ray diffraction diagram.

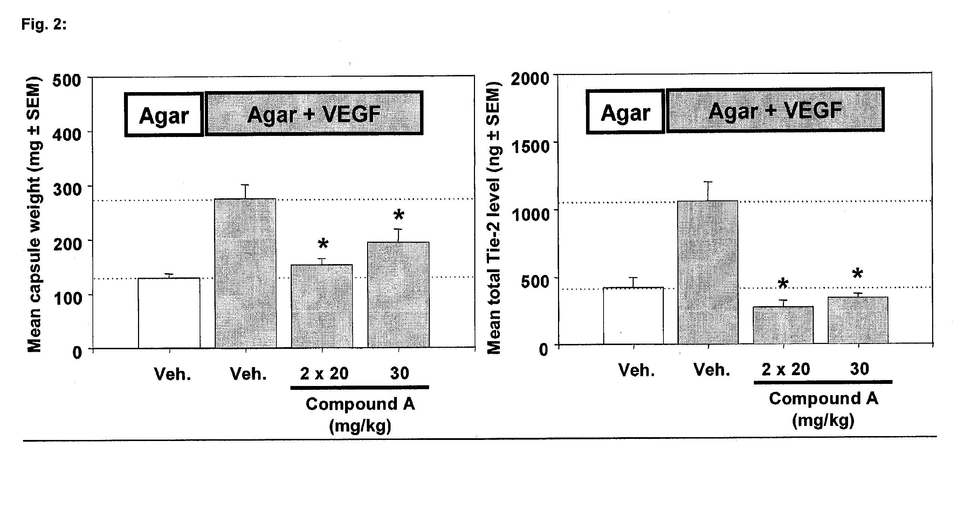

Fig. 2 shows the Effects of compound A on VEGF induced neo vascularization in vivo. FVB mice implanted with Teflon agar chamber loaded with agar alone (agar) or with 2 mg/mL of VEGF165 (agar + VEGF) were treated orally at the indicated dose levels and regimen with compound A or with the vehicle control (Veh., all panels) for 4 days. Animals were sacrificed 24 h post last dose, for quantification of the amount of VEGF induced neo-vascularized tissue (left panel), and Tie-2 levels by ELISA (right panel). *, p = < 0.05 (ANOVA - Dunnett's) over VEGF treated controls.

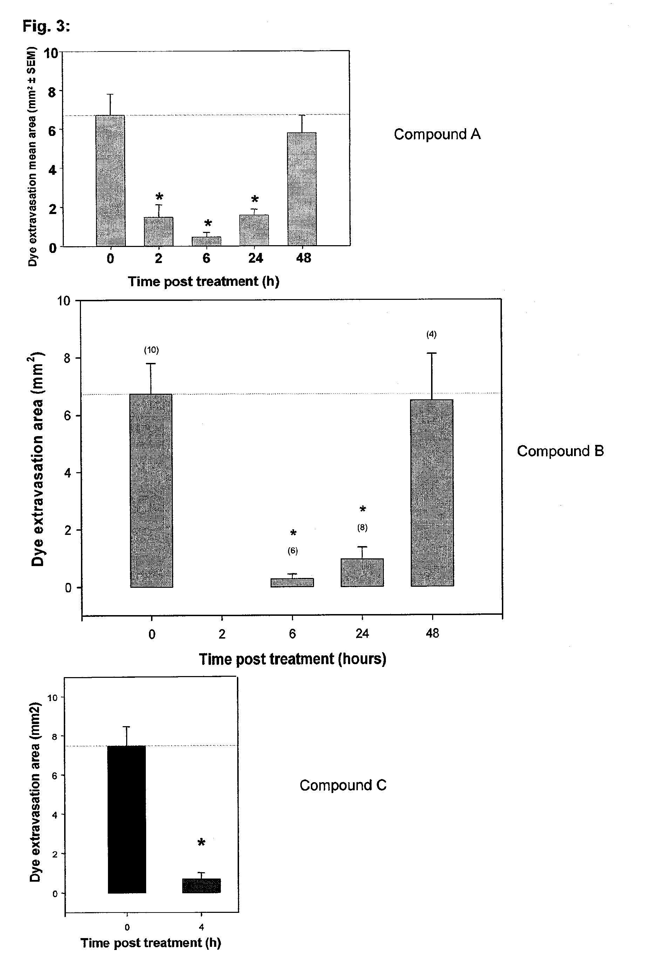

Fig. 3: Effects of compound A, B and C on VEGF induced permeability in vivo. FVB mice pre-treated orally for the indicated time either with Compound A (30 mg/kg), or Compound B (7.5 mg/kg), or Compound C (50 mg/kg) were injected i.v. with Evans blue and challenged 30 minutes later with VEGF injection in the ear. Mice were then sacrificed, the dye extravasation area measured. *, p = < 0.05

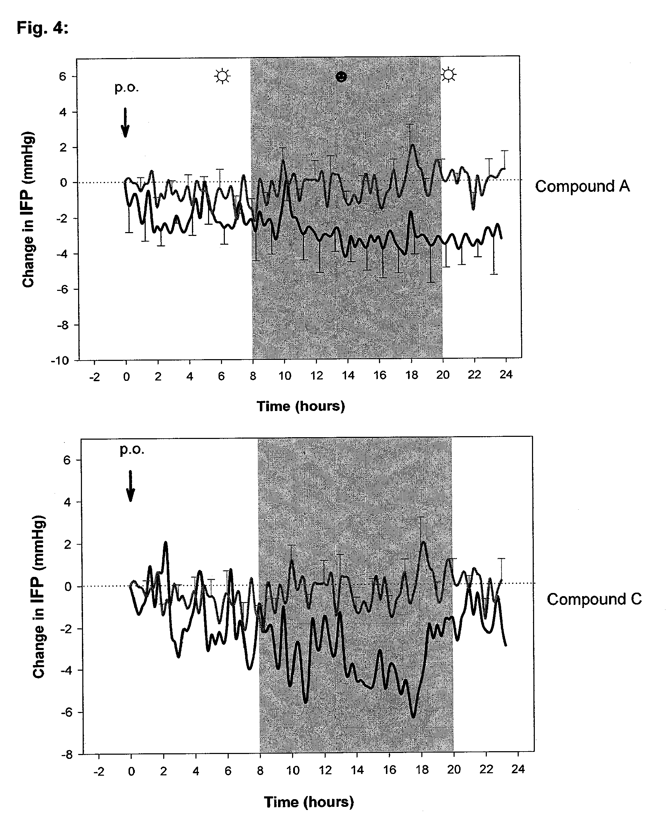

Fig. 4: Effects of compound A and C on tumor intrafluid pressure. Orthotopic BN472 tumor bearing rats containing a pressure sensing catheter inserted in the tumor were treated orally, once either with Compound A (30 mg/kg - upper panel, dark lane), or Compound C (2.5 mg/kg -bottom panel - dark lane), or with the vehicle control (both panels - grey lane) . The IFP was recorded for 24 h and variations (delta) plotted (top panel, dark lines: untreated animals; grey line/ applied treatment as indicated above the graph)

### Examples

The efficacy of the compounds of formula (I) and (II) and salts thereof can be demonstrated as follows:

### Example 1: HUVEC proliferation assay:

The effects on VEGF-induced proliferation of HUVEC were tested using a BrdU incorporation kit (Biotrak Cell Proliferation Elisa System V.2, Amersham, England). Sub-confluent HUVEC were seeded at a density of 5 x 10³ cells per well into 96-well plates coated with 1.5% gelatin and then incubated at 37 °C and 5 % CO₂ in growth medium with 5% FBS (PromoCell, Switzerland). After 24 h, the medium was replaced by basal medium containing 1.5 % FBS. After another 24 h, the medium was renewed and compound or vehicle control added. Human VEGF₁₆₅ (10 ng/ml) was added at the same time. After 24 h of incubation, BrdU labeling solution was added and cells incubated for additional 24 h before fixation, blocking and addition of peroxidase-labeled anti-BrdU antibody. Bound antibody was then detected using 3,3'5,5'-tetramethylbenzidine substrate, which forms a coloured reaction product that is quantified with a spectrophotometer at 450 nm.

### Example 2: In vivo chamber implant angiogenesis assay

Sterile tissue chambers made of perfluoro-alkoxy-Teflon® were filled with 500 µl molten 0.8% w/v agar containing 20 U/mL heparin (Novo Nordisk A/S, Bagsvaerd, Denmark) with or without growth factor (VEGF₁₆₅, 2 µg/mL) and implanted aseptically s.c. on the dorsal flank of female mice (FVB; Charles River Laboratories, les Oncins, France). Treatments started 4 to 6 hours before chamber implantation and then every day at the indicated dosage regimen for 3 days. The chambers were then recovered from the animals, and the vascularized tissue that had formed was removed and weighed. Tissue samples were homogenized in RIPA buffer (50 mM Tris-HCl, 121 mM NaCl, 1 mM EDTA, 6 mM EGTA, 1% NP-40, 20 mM NaF, 1 mM Pefabloc SC, 1 mM Na₃VO₄), centrifuged for 1 h at 7000 rpm, and the supernatant filtered using a 0.45 µm syringe filter (Acrodisc® GF, Gelman Sciences, Ann Arbor, MI, USA). For Tie-2 level determination, Nunc (Naperville, IL) Maxisorp 96-well plates were coated over night at 4°C with the capture antibody, anti-Tie-2 AB33 (UBI, Hauppauge, NY), with a concentration of 2 µg/mL (100 µL/well). Wells were washed in TPBS (Tween 80 PBS) and blocked by incubating with 3% Top-Block (Juro, Lucerne, Switzerland) for 2 hours at room temperature. 300 µg of protein lysates were added and incubated for 2 hours, and the wells washed three times before addition of a goat anti-mouse Tie-2 antibody (R&D Systems, Minneapolis, MN; 0.5 µg/mL) and an alkaline phosphate conjugated anti-goat antibody (Sigma, St. Louis, MO; diluted 1:6,000) in TPBS + 0.1%Top-Block for 1 hour at room temperature. Tie-2 antibody complexes were detected after incubation with p-nitrophenyl phosphate substrate (Sigma). The absorbance of the spectro-photometric reaction was determined with an ELISA reader at 405 nm. Recombinant human extracellular domain of Tie-2 fused to the constant regions of human IgG1 (sTie-2Fc) dissolved in RIPA buffer was used as standard in a concentration range from 0.1 to 300 ng/well

### Example 3: In vivo VEGF induced permeability assay (Miles assay)

200 µl of Evans blue (0.5 %) was injected into the tail vein of female FVB mice. Thirty minutes later, the mice were anaesthesized (3% Isoflurane in O₂, Forene^{®}, Abbott AG, Cham, Switzerland) and then placed on an operating field maintained at a temperature of 39°C. Their ears were extended over a steel cone fitted with a double-sided sticker to expose the dorsal surface. With the aid of a microscope, a 30G hypodermic needle was then inserted in the skin between the first and second neurovascular bundle of the ear and tunnelled for 4-5 mm. Two microliters of VEGF₁₆₄ (10 ng/µl) were injected using a microliter syringe (250 µl, Hamilton, Bonaduz, Switzerland) forming a 2 x 2 mm sub-dermal blister. Evans-blue dye bound to serum albumin will extravasate at sites of increased microvascular permeability, generating a visible blue spot which provide a measure of vascular permeability. The site of intra-dermal injection was photographed 30 min after injection in all animals. VEGF-mediated vessel leakage is quantified by measurement of the area (mm²) of dye that extravasated at the site of VEGF injection using pixel-based threshold in a computer-assisted image analysis software (KS-400 3.0 imaging system, Zeiss, Germany).

### Example 4: Tumor interstitial fluid pressure

The IFP of BN472 tumors was measured in conscious, freely moving rats maintained in their home cage using an adapted fully implantable miniaturized radio-telemetry system composed of 4 basic components: an implantable transmitter (AM unit, model TLM-PAC10, volume: 1.1 cc, weight: 1.4 g) which continuously senses and transmits information from within the animal, one receiver located under the home cage, a matrix interface for coordination of signals and a computer-based data acquisition system for collection, analysis and storage of data. The body of the transmitter was implanted s.c. aseptic conditions into the flank of the animal under isofluorane anaesthesia (3% Isoflurane in O₂). Briefly, once animals are fully unconscious, they are placed on a heated blanket and the abdominal area is shaved. The ventral surface of the abdomen is then prepared by swabbing the skin with pevidine iodine surgical scrub. With the animal supine, a skin incision of approximately 30 mm is made along the midline of the abdomen and the skin separated from the muscle wall building an air pocket. The sterile transmitter is then positioned in this pocket and the sensing catheter tunnelled subcutaneously towards the tumor site (lowest mammary fat pad). The sensing pressure catheter was inserted into the body of the tumor (4-5 mm depth) and secured at the site of entry with tissue adhesive (Vetbond; 3M Company). Fluid communication between the tip of the pressure catheter and the tumor was tested by gentle compressing and decompressing the tubing connected to the telemetry transducer using a clamp. The catheter implantation was quoted as good if the readings before and after this test did not differed by more than 1 mm Hg. However, in most of the implanted tumors, intra-tumoral IFP pulse pressure waveform curves could be recorded unstintingly with high resolution confirming even more strongly the adequate sensing catheter placement. The total operation time for the implantation of the telemetry transmitter was about 20 min. Postoperative analgesia was provided using Buprenorphin (Temgesic^{®}, Reckitt and Colman) injection of 0.05 mg/kg s.c. twice, immediately after surgery and 8-12 h later. Following surgery the unconscious animal is placed on soft material in a clean cage with water ad *libitum.* An external heat lamp is used to maintain body temperature. The animals were allowed a period of 2 days to recover from surgery before starting acquisition of any physiological data. IFP was recorded continuously in all animals over 24 hours up to 10 days, and analysed in 1-min cyclic runs for 10 sec, with a 500-Hz sampling rate. Areas under the curve (AUC) recorded for 24 h post treatment was determined by using the trapezoidal rule method.

## Claims

1. Use of a compound of formula (II), 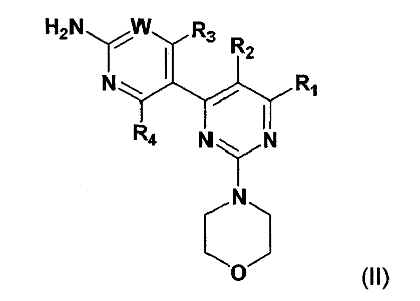 or a stereoisomer, tautomer, or pharmaceutically acceptable salt thereof wherein W is CR_{w} or N, wherein R_{w} is selected from the group consisting of:
(1) hydrogen,
(2) cyano,
(3) halogen,
(4) methyl,
(5) trifluoromethyl,
(6) sulfonamido;
R₁ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₁ₐ,
(13) -CO₂R₁ₐ,
(14) -CONR₁ₐR_{1b},
(15) -NR₁ₐR_{1b},
(16) -NR₁ₐCOR_{1b},
(17) -NR₁ₐSO₂R_{1b},
(18) -OCOR₁ₐ,
(19) -OR₁ₐ,
(20) -SR₁ₐ,
(21) -SOR₁ₐ,
(22) -SO₂R₁ₐ, and
(23) -SO₂NR₁ₐR_{1b},
wherein R₁ₐ, and R_{1b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl;
R₂ is selected from the group consisting
(1) hydrogen,
(2) cyano,
(3) nitro,
(4) halogen,
(5) hydroxy,
(6) amino,
(7) substituted and unsubstituted alkyl,
(8) -COR₂ₐ, and
(9) -NR₂ₐCOR_{2b},
wherein R₂ₐ, and R_{2b} are independently selected from the group consisting of
(a) hydrogen, and
(b) substituted or unsubstituted alkyl;
R₃ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₃ₐ,
(13) -NR₃ₐR_{3b},
(14) -NR₃ₐCOR_{3b},
(15) -NR₃ₐSO₂R_{3b},
(16) -OR₃ₐ,
(17) -SR₃ₐ,
(18) -SOR₃ₐ,
(19) -SO₂R₃ₐ, and
(20) -SO₂NR₃ₐR_{3b},
wherein R₃ₐ, and R_{3b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl; and
R₄ is selected from the group consisting of
(1) hydrogen, and
(2) halogen
for the manufacture of a pharmaceutical preparation for the treatment of a VEGF-driven angiogenic disease.

2. Use of a compound of formula (I), 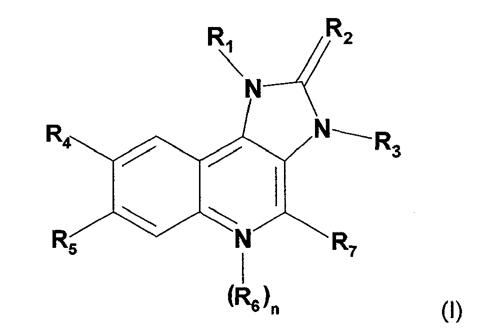 or a tautomer thereof, or a pharmaceutically acceptable salt, or a hydrate or solvate thereof, wherein
R₁ is naphthyl or phenyl wherein said phenyl is substituted by one or two substituents independently selected from the group consisting of Halogen; lower alkyl unsubstituted or substituted by halogen, cyano, imidazolyl or triazolyl; cycloalkyl; amino substituted by one or two substituents independently selected from the group consisting of lower alkyl, lower alkyl sulfonyl, lower alkoxy and lower alkoxy lower alkylamino; piperazinyl unsubstituted or substituted by one or two substituents independently selected from the group consisting of lower alkyl and lower alkyl sulfonyl; 2-oxo-pyrrolidinyl; lower alkoxy lower alkyl; imidazolyl; pyrazolyl; and triazolyl; R₂ is O or S; R₃ is lower alkyl; R₄ is pyridyl unsubstituted or substituted by halogen, cyano, lower alkyl, lower alkoxy or piperazinyl unsubstituted or substituted by lower alkyl; pyrimidinyl unsubstituted or substituted by lower alkoxy; quinolinyl unsubstituted or substituted by halogen; quinoxalinyl; or phenyl substituted with alkoxy; R₅ is hydrogen or halogen; n is 0 or 1; R₆ is oxido; with the proviso that if n=1, the N-atom bearing the radical R₆ has a positive charge; R₇ is hydrogen or amino;
for the manufacture of a pharmaceutical preparation for the treatment of a VEGF-driven angiogenic disease.

3. The use according to claim 1, where the compound of the formula (II) is 5-(2,6-di-morpholin-4-yl-pyrimidin-4-yl)-4-trifluoromethyl-pyridin-2-ylamine.

4. The use according to claim 2, where the compound of the formula (I) is 2-methyl-2-[4-(3-methyl-2-oxo-8-quinolin-3-yl-2,3-dihydro-imidazo[4,5-c]quinolin-1-yl)-phenyl]₋propionitrile or 2-methyl-2-[4-(3-methyl-2-oxo-8-quinolin-3-yl-2,3-dihydro-imidazo[4,5-c]quinolin-1-yl)-phenyl]-propionitrile or 8-(6-methoxy-pyridin-3-yl)-3-methyl-1-(4-piperazin-1-yl-3-trifluoromethyl-phenyl)-1,3-dihydro-imidazo[4,5-c]quinolin-2-one.

5. The use according to any one of claims 1 to 4, wherein the disease to be treated is Rheumatoid arthritis, Synovitis, Bone and cartilage destruction, Osteomyelitis, Pannus Growth, Osteophyte formation, Hepatitis, Pneumonia, Glomerulonephritis, Asthma, Nasal polyps, Transplantation, Liver generation, Retinopathy of prematurity, Age macular degeneration, Diabetic retinopathy, Chroidal and other intraocular disorders, Leukomalacia, Thyroiditis, Thyroid enlargement, Lympopholiferative disorders, Karposi's sarcoma, Haematologic malignacies (e.g., haemangiomas), Obesity, Spinal cord injury, Acutemyocardial infarction, Pulmonary, cerbral and retinal oedema, or further any combinations thereof.

6. A compound of formula (II) or formula (I) according to any one of claims 1 to 4 for use in the treatment of a VEGF-driven angiogenic disease.

7. A compound according to claim 6, wherein the disease to be treated is a disease according to claim 5.

8. A compound according to claim 6, wherein the compound of formula (II) or formula (I) is used on combination with at least one other active compound.

9. A compound according to claim 8, wherein the at least one other active compound is a VEGF or VEGFR targeting agent selected from the group consisting of Bevacizumab, anti-VEGF, Ranibizumab AVE0005, anti-VEGF HuMV833, anti-VEGF 2C3, anti-VEGF CBO-P11, Sutent, Sorafenib, Vatalanib, Zactima, Midostaurin, Angiozyme, AG-013736, Lestautinib, CP-547,632, CEP-7055, KRN633, NVP-AEE788, IMC-1211, ZK260253, Semaxanib, E-7107, AS-3, Cand5 and PTC-299; and the HSP90 inhibitors CNF1010, CNF2024, tanespimycinm, alvespimycin, IPI504, SNX5422 and NVP-AUY922.

10. A pharmaceutical preparation for the treatment of a VEGF-driven angiogenic disease comprising a compound of formula (II) or formula (I), according to any one of claims 1 to 4, or a pharmaceutically acceptable salt thereof and a pharmaceutically acceptable carrier.

11. The pharmaceutical preparation according to claim 10, wherein the disease to be treated is a disease according to claim 5.

12. Use of a compound of formula (II) or formula (I), according to any one of claims 1 to 4, or a pharmaceutically acceptable salt thereof for the treatment of a VEGF-driven angiogenic disease.

13. The use according to claim 12, wherein the disease to be treated is a disease according to claim 5.

14. Combination of a compound of formula (II) or formula (I), according to any one of claims 1 to 4, or a pharmaceutically acceptale salt thereof and a VEGF or VEGFR targeting agent Bevacizumab, anti-VEGF, Ranibizumab AVE0005, anti-VEGF HuMV833, anti-VEGF 2C3, anti-VEGF CBO-P11, Sutent, Sorafenib, Vatalanib, Zactima, Midostaurin, Angiozyme, AG-013736, Lestautinib, CP-547,632, CEP-7055, KRN633, NVP-AEE788, IMC-1211, ZK260253, Semaxanib, E-7107, AS-3, Cand5 and PTC-299; and the HSP90 inhibitors CNF1010, CNF2024, tanespimycinm, alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of a VEGF-driven angiogenic disease.

15. Combination according to claim 14, wherein the compound of formula (II) is 5-(2,6-di-morpholin-4-yl-pyrimidin-4-yl)-4-trifluoromethyl-pyridin-2-ylamine (Compound C).
